# EUROPÄISCHE PATENTSCHRIFT

(11) **EP 1 989 523 B1**
(45) Veröffentlichungstag und Bekanntmachung des Hinweises auf die Patenterteilung: **24.02.2010**
(21) Anmeldenummer: 07711654.9
(22) Anmeldetag: 23.02.2007
(51) Int. Cl.: G01L 19/14, A61M 5/168

(54) **VERFAHREN ZUM HERSTELLEN EINER FLUIDDRUCKMESSEINHEIT UND KOMPONENTE ZUM EINSATZ IN EINER FLUIDDRUCKMESSEINHEIT**
METHOD FOR PRODUCING A FLUID PRESSURE GAUGE AND COMPONENT FOR USE IN A FLUID PRESSURE GAUGE
PROCÉDÉ DE FABRICATION D'UNE UNITÉ DE MESURE DE LA PRESSION D'UN FLUIDE, ET COMPOSANT DESTINÉ À ÊTRE UTILISÉ DANS LADITE UNITÉ.

(30) Priorität: 24.02.2006 DE 102006008752
(43) Veröffentlichungstag der Anmeldung: 12.11.2008
(73) Patentinhaber: Smiths Medical Deutschland GmbH, 85614 Kirchseeon (DE)
(72) Erfinder: BECK, Bernd, 72414 Rangendingen (DE); WEBER, Jörg, 83533 Edling (DE)
(74) Vertreter: Schenk, Markus
(86) Internationale Anmeldenummer: PCT/EP2007/001592
(87) Internationale Veröffentlichungsnummer: WO 2007/098893

(56) Entgegenhaltungen:
- EP-A- 0 360 286
- EP-A- 0 949 494
- EP-A2- 0 232 142
- DE-C1- 4 400 941
- US-A- 5 848 971

## Beschreibung

Die vorliegende Erfindung bezieht sich auf das technische Gebiet der Fluiddruckmessung und insbesondere auf die Herstellung einer Komponente einer Fluiddruckmesseinheit, wie z.B. solchen zur Blutdruckmessung durch Druckübertragung unter Zuhilfenahme einer Flüssigkeitssäule, wie der Flüssigkeit in einem Druckmessschlauch bzw. einer Druckmessleitung.

Um einen möglichst geringen Aufwand in Krankenhäusern oder Kliniken bei der Patientenversorgung zu verursachen, besteht zunehmendes Interesse darin, verschiedene Mess- und Diagnosesysteme miteinander zu kombinieren, um hierdurch einerseits auf die Scheu von Patienten vor einem übermäßigen Technikeinsatz einzugehen und andererseits auch eine bessere Übersichtlichkeit der medizinischen Apparaturen für das medizinische Personal zu gewährleisten. Insbesondere bestehen starke Bestrebungen dahingehend, die herkömmlicherweise verwendete Vielzahl von Kabeln und Leitungen zu reduzieren, um eine bessere Übersichtlichkeit der einzelnen Leitungen zum oder vom Patienten zu gewährleisten, um insbesondere auch eine mögliche falsche Bediendung der medizinischen Systeme durch ein "Vertauschen" der Kabel und Leitungen weitgehend auszuschließen. In diesem Zusammenhang wurde bereits eine Blutdruckmessung in ein Infusionssystem integriert, wobei ausgenutzt wurde, dass die Flüssigkeitssäule vom arteriellen oder venösem Katheter (nicht gezeigt) zu einem Druckaufnehmer als Druckübertragungsmedium verwendet werden kann, wenn ein ausreichend formstabiler Schlauch zwischen venösem/arteriellem Katheter und Druckaufnehmer verwendet wird. Der Blutdruck wird dann über die Infusionslösung von einer Arterie oder Vene über den Katheter und über den genannten formstabilen Schlauch zu einem speziellen Sensor übertragen, der ausgebildet ist, um Druckschwankungen in der Infusionslösung zu erkennen, die dann den zu messenden Blutdruckwerten entsprechen. Ein solcher Drucksensor wird auch Transducer genannt.

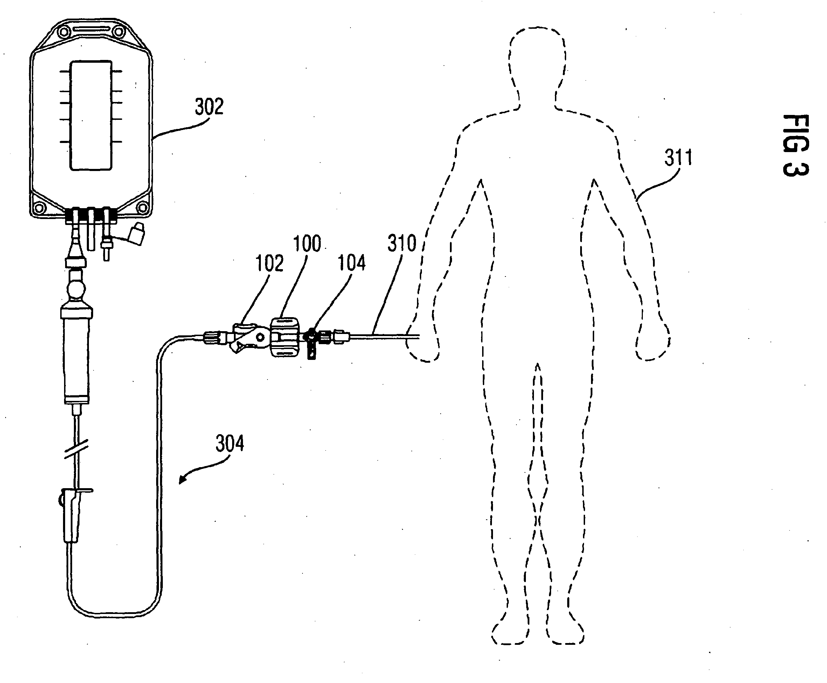

Der prinzipielle Aufbau eines derartigen Infusionssystems mit der Funktionalität einer Blutdruckmessung ist in Fig. 3 dargestellt. Ein solches System weist einen Infusionslösungsbeutel 302 auf, der über einen Infusionslösungsschlauch 304 mit einem ersten Hahn oder Spülsystem 102 verbunden ist. Das Spülsystem ist mit einer Kapillare versehen, die als Druckentkoppelung der weiteren Systemkomponenten vom Infusionslösungsbeutel 302 dient. Das Spülsystem 102 kann dabei derart entworfen sein, dass es eine vordefinierte Durchflussrate von beispielsweise 3 ml pro Stunde gewährleistet.

Der erste Hahn bzw. das Spülsystem 102 ist mit einem Transducer 100 verbunden, der wiederum mit einem zweiten Hahn 104 verbunden sein kann. Der zweite Hahn 104 ist über eine druckstabile Druckmessleitung 310 (beispielsweise über einen Katheter) mit dem Patienten 311 verbunden. Hierbei ist anzumerken, dass die in Fig. 3 dargestellten Druckmessleitung 310 eine hohe Formstabilität, d.h. eine geringe Elastizität, aufweisen sollte, um Druckschwankungen nicht zu verfälschen oder zu dämpfen.

Der zweite Hahn 104 wird normalerweise zum "Nullen", d.h. zum Einstellen oder Kalibrieren des am Transducer 100 zu messenden Drucks gegenüber atmosphärischem Druck verwendet, indem bei von dem Patienten entkoppelten Zustand der Hahn 104 des Transducers 100 mit atmosphärischem Druck beaufschlagt wird.

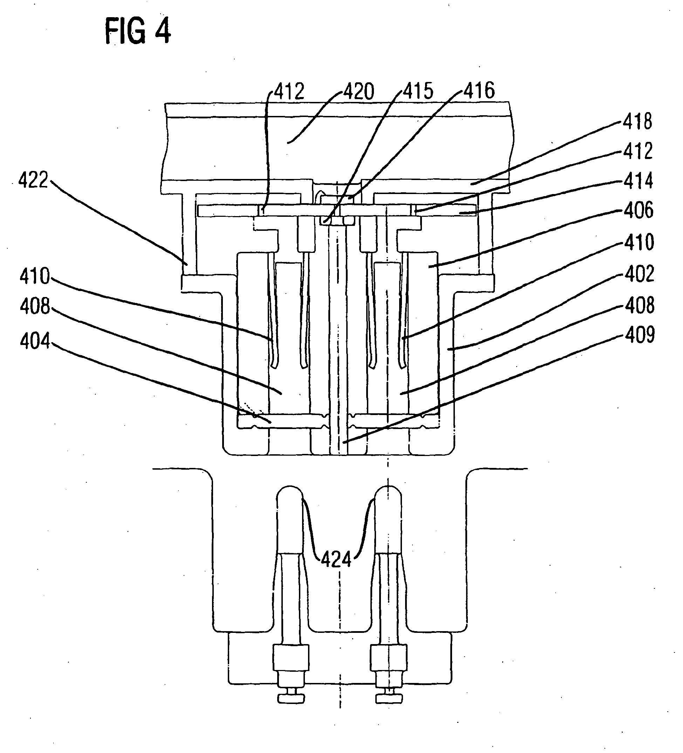

Ein bekannter Typ von Transducer in Druckmesssystemen besteht aus mehreren Einzelteilen. Ein typischer Aufbau eines derartigen herkömmlichen Transducers ist in der Querschnittsdarstellung in Fig. 4 wiedergegeben.

Gemäß der Fig. 4 weist der herkömmliche Transducer ein Gehäuseunterteil 402 (beispielsweise in Becherform) auf, in dem eine vorgefertigte Membran 404 (beispielsweise aus Silikon) eingelegt ist. Auf die Membran 404 ist ein Kontaktträger 406 aufgesetzt, der Öffnungen 408 aufweist, in die Buchsenkontakte 410 mit Kontaktfedern eingepresst sind.

Ferner weist das Gehäuseunterteil 402, die Membran 404 und der Kontaktträger 406 ein Mittelloch 409 auf, durch das ein atmosphärischer Druck der Außenumgebung des Transducers an einen oberen Bereich des Kontaktträgers 406 anliegt.

Die Kontaktfedern sind an Anschlusskontakte 412 auf der Keramikplatte 414 angelötet, wobei die Kontaktanschlüsse 412 mit Kontakten eines Messchips 416 verbunden sind. Der Messchip 416 ist dabei auf einer ersten Seite der Keramikplatte 414 über einer Öffnung in der Keramikplatte 414 angeordnet, die Teil des Mittelloches 409 ist, wohingegen die Anschlusskontakte 412 auf einer der ersten Seite gegenüberliegenden zweiten Seite der Keramikplatte 414 angeordnet sind.

Weiterhin ist zwischen dem Kontaktträger 406 und der Keramikplatte 414 ein O-Ring 415 angeordnet, der die Öffnung 409 in dem Kontaktträger 406 lateral umgibt. Hierdurch liegt zum atmosphärischen Druckausgleich über das Mittelloch 409 bzw. die Öffnung in der Keramikplatte 414 ein Umgebungsluftdruck an dem Messchip 416 als Referenzwert an. Weiterhin wird durch den O-Ring 415 eine Abdichtung der Buchsenkontakte bzw. der elektrischen Verbindungen wie der Anschlusskontakte 412 oder Leiterbahnen auf der Keramikplatte 414 gegenüber leitfähigen Flüssigkeiten, wie beispielsweise der Infusionslösung oder von außen anliegenden Flüssigkeiten, geliefert, die gegebenenfalls bei Anschließen des Transducers in das Innere des Gehäuses eindringen könnten.

Ferner weist der Transducer ein Gehäuseoberteil 418 auf, das einen Durchflusskanal 420 für die Infusionslösung umfasst. Der Messchip 416 ist dabei in einer Ausnehmung des Durchflusskanals 420 angeordnet. Zusätzlich ist der Messchip mit einem Gel abgedeckt, das den Messchip 416 gegenüber der Infusionslösung schützt, da bei direktem Kontakt der elektrisch leitfähigen.Infusionslösung mit dem Messchip 416 eine Verfälschung der Messergebnisse durch "Kriechströme" in der elektrisch leitfähigen Infusionslösung auftreten würde. Über das Gel können jedoch Druckschwankungen in dem Fluid an den Messchip 416 übertragen werden. Weiterhin ist das Gehäuseoberteil 418 an Schweiß- oder Klebestellen 422 mit dem Gehäuseunterteil 402 verbunden (beispielsweise über eine Ultraschallschweißung), so dass der Transducer in seiner Endform hergestellt wird.

Ein Anschließen des Transducers erfolgt nun derart, dass ein Stecker bzw. eine Steckverbindung 424 über eine Öffnung in den Boden des Gehäuseunterteils 402 durch die Membran 404 hindurchgeführt und elektrisch leitfähig mit den Kontaktfedern der Buchsenkontakte 410 in Verbindung gebracht wird. Hierzu ist beispielsweise die Membran 404 bereits mit Schlitzen versehen, welche bei Einstecken der Verbindungsstecker 424 eventuelle Flüssigkeiten an den Steckerstiften abstreifen.

Aufgrund der hohen Anzahl von Einzelteilen ist der Fertigungsprozess für den in Fig. 4 dargestellten Typ von Transducer aufwendig und kostenintensiv. Ein möglicher Fertigungsprozess umfasst dabei beispielsweise folgende Schritte.

Zunächst werden bei der Fertigung eines Transducers die Buchsenkontakte 410 in den Kontaktträger 406 eingepresst. Anschließend wird der O-Ring 415 auf diesen mit den Buchsenkontakten 410 versehenen Kontaktträger 406 aufgesetzt, woraufhin die Keramikplatte 414 ( Hybridkeramik mit aufgedruckten Leiterbahnen und Widerständen) auf den O-Ring 415 aufgelegt wird. An der Keramikplatte 414 ist der Messchip 416 mit den entsprechenden Anschlusskontakten 412 angebracht. Nach dem Aufsetzen der Hybridkeramik auf den O-Ring 415 und den Kontaktträger 406 werden Lötfahnen 411 der Buchsenkontakte 410, die in den Kontaktträger eingepresst sind, mit den Anschlusskontakten 412 der Hybridkeramik verbunden und verlötet. Aufgrund der beim Löten auftretenden hohen Temperaturen muss der Kontaktträger 414 aus einem speziellen temperatur- und formstabilen Material gefertigt werden, was kostenintensiv und aufwendig ist und zudem die Auswahl der Materialkandidaten im Hinblick auf andere gewünschte Materialeigenschaften einschränkt. Nach der Kontaktierung wird die Membran 404, beispielsweise als Dichtmembran aus Silikonmaterial, in das Gehäuseunterteil 402 eingesetzt, um zwischen dem Kontaktträger 406 und dem Gehäuseunterteil 402 eingeklemmt zu werden. Dadurch werden die in den Kontaktträger 406 eingepressten Buchsenkontakte 410 zusätzlich von unten abgedichtet. Die Membran 404 ist dabei eine vorgefertigte Silikonmembran, die als Einzelteil bei der Herstellung eines derartigen Transducers verwendet wird. Hiernach erfolgt ein Zusammensetzen des Gehäuseunterteils 402 mit einem Gehäuseoberteil 418, wodurch die Membran 404 wie oben beschrieben eingeklemmt wird. Das Gehäuseunterteil 402 kann mittels Ultraschall an der Schweißstelle 422 mit dem Gehäuseoberteil 418 verschweißt werden oder aber auch mit Klebstoff oder Lösungsmittel mit dem Gehäuseoberteil 418 verbunden werden. Das Verschweißen mittels Ultraschall ermöglicht zwar eine hohe Dichtheit der Verbindung, ist aber sehr aufwendig, da auf eine dichte Verbindung von Gehäuseoberteil 418 und Gehäuseunterteil 402 des Transducers geachtet werden muss, damit keine Flüssigkeit in das Transducergehäuse eindringen kann. Dies könnte nämlich zu einer elektrischen Verbindung zwischen den Kontakten und zu einer Drift des Druckmesssignals führen. Daher ist es nötig, eine besondere Prüfung der Dichtheit zwischen Gehäuseoberteil 418 und Gehäuseunterteil 402 durchzuführen, was aufwendig und insbesondere bei der Massenfertigung von derartigen Transducern teuer ist. Schließlich wird noch ein Gel in die Ausnehmung des Durchflusskanals 420 eingefüllt, um den Sensor 416 gegenüber Infusionslösung fluiddicht abzudichten, jedoch gleichzeitig eine Druckübertragung von dem Fluid in dem Durchflusskanal 120 auf den Sensor 416 zu ermöglichen. Dieses Einfüllen kann jedoch aus herstellungstechnischen Gründen nur mittels eines aufwändigen Verfahrens unter Zuhilfenahme einer Spritze durch den Durchflusskanal 120 erfolgen.

Bekannte Verfahren und Komponenten werden in EP 360286 A, US 5 848 971 A, DE 4400941C, EP 949494 A und EP 232 142 A offenbart.

Die Aufgabe der vorliegenden Erfindung besteht somit darin, eine Komponente zum Einsatz einer Fluiddruckmesseinheit und ein Verfahren zum Herstellen einer Komponente einer Fluiddruckmesseinheit zu schaffen, die eine kostengünstigere bzw. weniger aufwendige Herstellungsweise ermöglichen.

Diese Aufgabe wird durch ein Verfahren zum Herstellen einer Komponente einer Fluiddruckmesseinheit gemäß Anspruch 1 sowie einer Komponente zum Einsatz in einer Fluiddruckmesseinheit gemäß Anspruch 10 gelöst.

Das erfindungsgemäße Verfahren zum Herstellen einer Komponente einer Fluiddruckmesseinheit umfasst folgende Schritte:
Bereitstellen eines Messelementes mit einem auf einer ersten Seite eines Chipträges angeordnete Messchip und einer Mehrzahl von buchsenförmigen Anschlusskontakten zur Kontaktierung von Kontaktanschlüssen des Messchips, wobei die buchsenförmigen Anschlusskontakte von einer der ersten Seite des Chipträgers gegenüberliegenden zweiten Seite des Chipträgers hervorstehen;
Bereitstellen eines Gehäuseelementes, das eine Mehrzahl von Aufnahmelöchern in einer ersten Seite des Gehäuseelementes aufweist, die ausgebildet sind, um bei einem Zusammenfügen des Gehäuseelementes mit dem Messelement die buchsenförmigen Anschlusskontakte aufzunehmen, wobei sich die Aufnahmelöcher von der ersten Seite des Gehäuseelementes aus zu einer der ersten Seite des Gehäuseelementes gegenüberliegenden zweiten Seite des Gehäuseelementes hin erstrecken,
wobei das Bereitstellen des Gehäuseelementes ein Anspritzen eines Verschlusselementes an die zweite Seite des Gehäuseelementes zum Verschließen der Aufnahmelöcher an der zweiten Seite des Gehäuseelementes umfasst; und
Zusammenfügen des Messelementes und des Gehäuseelementes derart, dass die zweite Seite des Chipträgers der ersten Seite des Gehäuseelementes gegenüber liegt und wobei die buchsenförmigen Anschlusskontakte in den Aufnahmelöchern angeordnet werden, um die Komponente der Flüiddruckmesseinheit herzustellen.

Die vorliegende Erfindung betrifft eine Komponente zum Einsatz in einer Fluiddruckmesseinheit, wobei die Komponente folgende Merkmale aufweist:
ein Gehäuseelement mit einer Mehrzahl von Aufnahmelöchern, die sich von der ersten Seite des Gehäuseelementes aus zu einer der ersten Seite des Gehäuseelementes gegenüberliegenden zweiten Seite des Gehäuseelementes hin erstrecken, wobei das Gehäuseelement auf der zweiten Seite ein Verschlusselement zum Verschließen der Aufnahmeöffnungen an der zweiten Seite des Gehäuseelementes aufweist, das einstückig mit dem Gehäuseelement verbunden ist, wobei sich ein Material des Verschlusselementes von einem Material des Gehäuseelementes unterscheidet; und
ein Messelement mit einem auf einer ersten Seite eines Chipträgers angeordneten Messchip und einer Mehrzahl von buchsenförmigen Anschlusskontakten zur Kontaktierung von Kontaktanschlüssen des Messchips, wobei die buchsenförmigen Anschlusskontakte von einer der ersten Seite des Chipträgers gegenüberliegenden zweiten Seite des Chipträgers hervorstehen, wobei das Messelement und das Gehäuseelement derart aufgebaut sind, dass das Messelement und das Gehäuseelement derart zusammenfügbar sind, dass in einem zusammengefügten Zustand die buchsenförmigen Anschlusskontakte in die Aufnahmelöcher hineinragen und die zweite Seite des Chipträgers der ersten Seite des Gehäuseelementes gegenüber liegt, um die Komponente zum Einsatz in der Fluiddruckmesseinheit zu bilden.

Der vorliegenden Erfindung liegt zunächst die Erkenntnis zugrunde, dass eine bedeutend einfachere bzw. kostengünstigere Herstellung ermöglicht wird, wenn die Notwendigkeit eines Verwendens eines kostenintensiven und temperatur- und formstabilen Materials für den Buchsenkontaktaufnehmer bzw. den Kontaktträger, in den Buchsenkontakte eingepresst werden, entfällt.

Gemäß einem Aspekt der vorliegenden Erfindung wird obige Erkenntnis um die Erkenntnis erweitert, dass es die freiere Auswahl des Materials für den Buchsenkontaktaufnehmer ermöglicht, Buchsenkontaktaufnehmer und Gehäuseunterteil einstückig auszubilden, da auch ein Anspritzen des Abdichtelementes (beispielsweise als Ersatz des O-Ringes) an die erste Seite des Gehäuseelementes und/oder ein Anspritzen eines Verschlusselementes (das beispielsweise die Silikonmembran ersetzt) an die zweite Seite des Gehäuseelementes möglich wird. Dies wiederum bedeutet eine deutliche Vereinfachung beim Zusammensetzen der herzustellenden Komponente der Fluiddruckmesseinheit, da in diesem Fall die Anzahl von zu verarbeitenden Einzelteilen deutlich reduziert werden kann. Dies resultiert daher, dass das Anspritzen des Abdichtelementes bzw. des Verschlusselementes verfahrenstechnisch und somit maschinell wesentlich effizienter ausgeführt werden kann als ein händisch (oder auch maschinell) durchgeführtes Zusammensetzen der Komponente der Fluiddruckmesseinheit aus einer Vielzahl von Einzelteilen. Ein Vermeiden eines separaten O-Ringes und der Membran auf der zweiten Seite des Gehäuseelementes ermöglicht daher zum Beispiel das Entfallen zumindest eines Herstellungsschritts beim Zusammensetzen der Komponente der Fluiddruckmesseinheit.

Anders ausgedrückt bietet der obige Ansatz den Vorteil, dass durch das Entfallen der Verwendung einer großen Zahl von Einzelteilen, wie beispielsweise des O-Ringes und/oder der Silikonmembran, eine Kostenreduktion aufgrund der Einsparung von Herstellungsschritten möglich ist Der untere Teil des Gehäuses kann als Gehäuseelement mit dem Abdichtelement und dem Verschlusselement einstückig hergestellt werden, wodurch sich einzelne Herstellungsschritte zur Herstellung bzw. Bereitstellung des Gehäuseelementes vermeiden lassen (wie beispielsweise das Einbringen des vorbestimmten Kontaktträgers in das Gehäuseunterteil). Ein derartiger Ansatz bietet den Vorteil, dass nunmehr nicht in einem eigenen aufwendigen Herstellungsschritt ein Dichtungsmembranplättchen in das Gehäuseunterteil passgenau eingebracht werden muss, auf den der Kontaktträger aufgesetzt wird, sondern dass die Dichtungsmembran zum Abdichten der Aufnahmelöcher im Gehäuseelement durch einfaches Anspritzen herstellbar ist. Hierdurch lässt sich wiederum eine Kostenreduktion durch die Verwendung eines einfacheren Herstellungsschrittes als einem Schritt des Vorfertigens der Dichtmembran und des Einbringen der vorgefertigten Dichtmembran realisieren.

Ein weiterer Aspekt ergänzt die oben zugrunde liegende Erkenntnis dadurch, dass erkannt worden ist, dass die freie Auswahl des Materials für den Buchsenkontaktaufnehmer bzw. des Gehäuseunterteils ermöglicht werden kann, wenn die Buchsenkontakte vor Einfügen der Buchsenkontakte in die Buchsenkontaktlöcher an der Keramikplatte befestigt werden, wodurch für das Gehäuseunterteil auch die Verwendung eines Materials für das Gehäuseelement möglich ist, das keine derart hohen Temperatur- und Formstabilitätsanforderungen nötig macht und dafür in anderer Hinsicht vorteilhafter ist, wie z.B. im Hinblick auf die Kosten bei der Verwendung von Materialien mit hoher Temperatur- und Formstabilität.

Gemäß einem bevorzugten Ausführungsbeispiel des erfindungsgemäßen Verfahrens umfasst der Schritt des Bereitstellens des Gehäuseelementes ein Bilden einer Mittelöffnung zwischen den Aufnahmelöchern, wobei sich die Mittelöffnung von der ersten Seite des Gehäuseelementes zu der zweiten Seite des Gehäuseelementes erstreckt, wobei der Schritt des Bereitstellens des Messelementes ein Bereitstellen einer Öffnung in dem Chipträger unter dem Messchip umfasst und wobei der Schritt des Zusammenfügens mittels des Messelementes mit dem Gehäuseelement derart ausgeführt wird, dass die Mittelöffnung mit der Öffnung des Chipträgers in Verbindung ist und der Messchip fluidisch mit einer Außenumgebung der Komponente der Fluiddruckmesseinheit verbunden ist. Hierdurch ist es möglich, eine Kalibrierung des Messchips mit einem Umgebungsluftdruck durchzuführen, sodass der Transducer in den verschiedenen Anwendungsszenarien vorteilhaft einsetzbar ist.

Auch kann der Schritt des Bereitstellens des Messelementes ein Anbringen eines Sensordomes auf der ersten Seite des Chipträgers umfassen, wobei der Sensordom den Messchip umgibt und wobei der Schritt des Bereitstellen ferner ein Befüllen des Sensordoms mit einem Gel umfasst, derart, dass der Messchip von dem Gel abgedeckt ist. Dies bietet den Vorteil, dass das Gel schon vor dem Zusammensetzen der Komponente der Fluiddruckmesseinheit oder zumindest vor dem Anschluss an den Durchlasskanal eingefüllt werden kann, und ein umständliches und daher aufwändiges Einfüllen des Gels auf den Messchip über den Fluidkanal nicht mehr erforderlich ist.

In einem weiteren bevorzugten Ausführungsbeispiel der vorliegenden Erfindung umfasst der Schritt des Bereitstellens des Gehäuseelementes das Anspritzen des Verschlusselementes, wobei das Bereitstellen des Gehäuseelementes ferner das Bilden von Kontaktierungslöchern in dem Verschlusselement im Bereich der Aufnahmelöcher umfasst. Die Kontaktierungslöcher können dabei als Durchgangslöcher ausgebildet sein. Das Vorsehen der Löcher bietet dabei den Vorteil, dass kein Vorschlitzen des Verschlusselementes und kein unkontrolliertes Aufreißen des Verschlusselementes beim Einstecken von Kontaktstiften zur Kontaktierung des Transducers zu befürchten ist. Weiterhin bietet eine ring- oder kreisförmige Ausgestaltung der Kontaktierungslöcher den Vorteil, dass durch derartige Formen die Kontaktstifte besser von kontaminierenden Flüssigkeiten wie z.B. Kochsalzlösung abgestreift und der Innenraum des Gehäuses besser abgedichtet werden können, so dass beispielsweise ein Eindringen von Flüssigkeiten wie der Infusionslösung in die Aufnahmelöcher bzw. die darin angeordneten buchsenförmigen Anschlusskontakte verhindert wird und somit auch keine Verfälschung der Messergebnisse durch Fehlerströme zu befürchten ist. Ein Sackloch bietet den Vorteil, dass der Innenraum der Öffnung mit den buchsenförmigen Anschlusskontakten beim Transport oder allgemein nach der Herstellung bis zum Erstgebrauch nicht verunreinigt wird. Weiterhin kann das Bilden des Loches nicht nur durch ein Schneiden oder ein Stanzen erfolgen, sondern auch eine direkte Formung während des Anspritzens ist möglich, was keine Mehrkosten bzw. keinen Extra-Schritt erfordert. Es kann ferner ein vorgeschlitztes Sackloch verwendet werden, das ringförmig abdichtet, wobei gleichzeitig aber der geschlitzte Teil im ungesteckten Zustand flüssigkeitsdicht ist.

In einem weiteren bevorzugten Ausführungsbeispiel umfasst der Schritt des Bereitstellens des Gehäuseelementes ein Ausbilden eines Basiskörpers mit den Aufnahmelöchern aus einem thermoplastischen Material, wie z.B. einem Polyolefin oder einem anderen für die Umspritzung mit einem zweiten Material geeigneten Kunststoff, und ein Anspritzen von TPE zum Ausbilden des Abdichtelementes und/oder des Verschlusselementes. Eine derartige Material-Kombination für die angespritzten Elemente und das Gehäuseelement bietet den Vorteil einer besonders gut haftenden Verbindung der angespritzten Elemente an dem Gehäuseelement.

Auch kann bei einem Ausführungsbeispiel des erfindungsgemäßen Verfahrens der Schritt des Bereitstellens ein Anspritzen eines Abdichtelementes umfassen, wobei der Schritt des Bereitstellens des Gehäuseelementes ferner das Anspritzen eines von dem Abdichtelement getrennten weiteren Abdichtelementes an die erste Seite des Gehäuseelementes umfasst. Beispielsweise kann auch der Schritt des Anspritzen ein Anspritzen einer Dichtlippe an ein Verbindungsbereich zwischen dem Gehäuseelement und einem Fluidkanal-Anschlusselement umfassen. Das Vorsehen einer derartigen angespritzten und vorzugsweise umlaufenden Dichtlippe zum Fluidkanal-Anschlusselement bietet dann den Vorteil einer konstruktiv einfachen Umsetzung einer Abdichtung zwischen einem oberen und einem unteren Teilelement der Fluiddruckmesseinheit, so dass beispielsweise ein aufwendiges Verschweißen per Ultraschall und eine nachfolgende gesonderte Prüfung nicht mehr notwendig sind. Dies kann sich insbesondere dann als vorteilhaft erweisen, wenn das obere Teilelement, d.h. das Fluidkanal-Anschlusselement, und das untere Teilelement, d.h. das Gehäuseelement, durch einfaches Zusammenpressen verbunden werden, wie es beispielsweise unter Verwendung einer Schnappvorrichtung möglich wäre. Auch kann entsprechend ein Schraubverschluss verwendet werden. Ziel einer derartigen Abdichtung ist es, das obere Teilelement gegen das untere Teilelement abzudichten, unabhängig von einer Abdichtung zum Fluidkanal. Hierdurch lassen sich, entweder mittels der Schnappverbindung oder mittels des Schraubverschlusses, einfache Befestigungsmöglichkeiten realisieren, die deutlich kostengünstiger als das herkömmliche Ultraschall-basierte Verschweißen sind. Zugleich wird jedoch auch sichergestellt, dass eine Dichtheit zwischen dem oberen und unteren Teilelement der Fluiddruckmesseinheit gewährleistet ist.

Das Fluidkanal-Anschlusselement kann einen Fluidkanal aufweisen, der auf einer Stelle im Bereich der Anschlussöffnung einen geringeren Querschnitt aufweist, als an einer weiteren Stelle, wobei im Bereich der Anschlussöffnung ein Sichtfenster in einer Wand des Fluidkanal angeordnet ist, das eine verbesserte Einsichtnahme auf den Messchip oder ein den Messchip umgebendes Gel von außerhalb der Fluiddruckmesseinheit ermöglicht. Eine derartige Querschnittsverteilung ermöglicht die Realisierung des Venturi-Prinzips, so dass das Fluid im Bereich der Anschlussöffnung schneller fließt als im Bereich des Fluidkanals an einer Stelle mit einem größeren Querschnitt. Insbesondere dann, wenn der Messsensor oder Messchip mit demjenigen Teil des Fluidkanals fluidisch verbunden ist, der einen geringeren Querschnitt als ein anderer Teilbereich des Fluidkanals aufweist, bietet eine derartige Anordnung den Vorteil, dass durch die erhöhte Durchflussgeschwindigkeit in dem mit dem Messsensor fluidisch verbundenen Teilbereich des Durchflusskanals sichergestellt werden kann, dass sich in diesem Teil keine Blasen mehr befinden, die sich möglicherweise beim erstmaligen Befüllen des Leitungssystems oder während des Gebrauchs an dem Messchip oder an Oberflächen oder Kanten in diesem Bereich abgesetzt haben. Um für einen Anwender die Möglichkeit bereitzustellen, eine bessere Kontrolle durchzuführen, ob sich tatsächlich keine Bläschen über dem Messchip abgesetzt haben, die ein Messergebnis verfälschen könnten, kann auch direkt über dem Messchip ein Sichtfenster oder eine Linse angeordnet sein, so dass ein Bereich des Durchflusskanals, der fluidisch mit dem Messchip oder einem Gel, das den Messchip umgibt verbunden ist, von außen vergrößert betrachtet werden kann.

Ein Ausführungsbeispiel der vorliegenden Erfindung wird nachfolgend anhand der beiliegenden Figuren näher erläutert. Es zeigen:
- Fig. 1A: eine Seitenansicht eines Teilbereichs eines Druckmesssystems, der ein Ausführungsbeispiel der erfindungsgemäßen Komponente einer Fluiddruckmesseinheit darstellt;
- Fig. 1B: eine Draufsicht der in Fig. 1A dargestellten Komponente einer Fluiddruckmesseinheit;
- Fig. 1C: eine Schnittansicht des in Fig. 1A und 1B dargestellten Ausführungsbeispiels der Komponente der Fluiddruckmesseinheit entlang der in Fig. 1B dargestellten Schnittlinie BA-BA in einer vergrößerten Darstellung;
- Fig. 1D: eine Schnittansicht des in Fig. 1B dargestellten Ausführungsbeispiels der erfindungsgemäßen Komponente einer Fluiddruckmesseinheit entlang der Schnittlinie BB-BB in einer vergrößerten Darstellung;
- Fig. 2: eine vergrößerte perspektivische Schnittdarstellung des Gehäuseunterteils bzw. des unteren Teilelementes der Fluiddruckmesseinheit aus Fig. 1A-1D;
- Fig. 3: eine schematische Darstellung eines Aufbaus eines Infusionssystems mit Druckmessung; und
- Fig. 4: eine Querschnittdarstellung eines herkömmlichen Transducers.

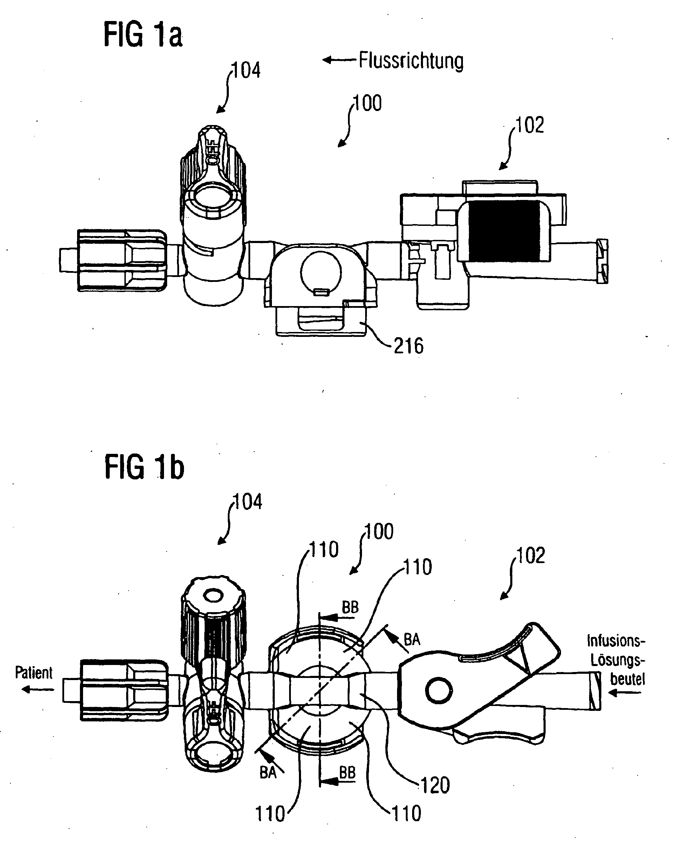

Die Fig. 1A zeigt einen Ausschnitt aus einem Druckmesssystem, das einen Transducer oder Druckaufnehmer 100 aufweist. Hierbei kann der Druckaufnehmer 100 derart in das Druckmesssystem eingebaut sein, dass er in Flussrichtung der Infusionslösung hinter einem Spülsystem (erster Hahn) 102 und vor einem zweiten Hahn 104 angeordnet ist. Das Spülsystem 102 kann zu einem Infusionslösungsreservoir hin angeordnet sein, während der zweite Hahn 104 beispielsweise zwischen dem Transducer 100 und einem Patienten geschaltet ist. Durch das Spülsystem 102 kann sichergestellt werden, dass der Transducer vor Druckschwankungen geschützt wird, die vom Infusionssystem herrühren, und dass eine gewünschte Durchflussrate sichergestellt ist, während der zweite Hahn 104 geschlossen werden kann, wenn Aktionen am Patienten, wie beispielsweise eine Blutabnahme durchgeführt werden. Außerdem ist der zweite Hahn zur "Nullung" des Drucksensors gegen Atmosphärendruck verwendbar.

Auch andere Ausführungsbeispiele mit beidseitigen Hähnen (102, 104) oder ganz ohne Hähne bzw. mit anderen Hahnanordnungen sind möglich.

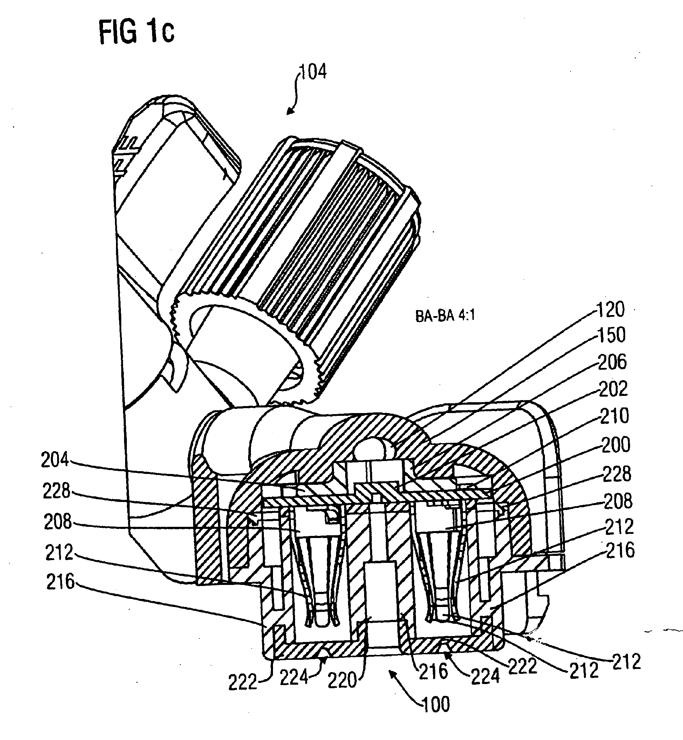

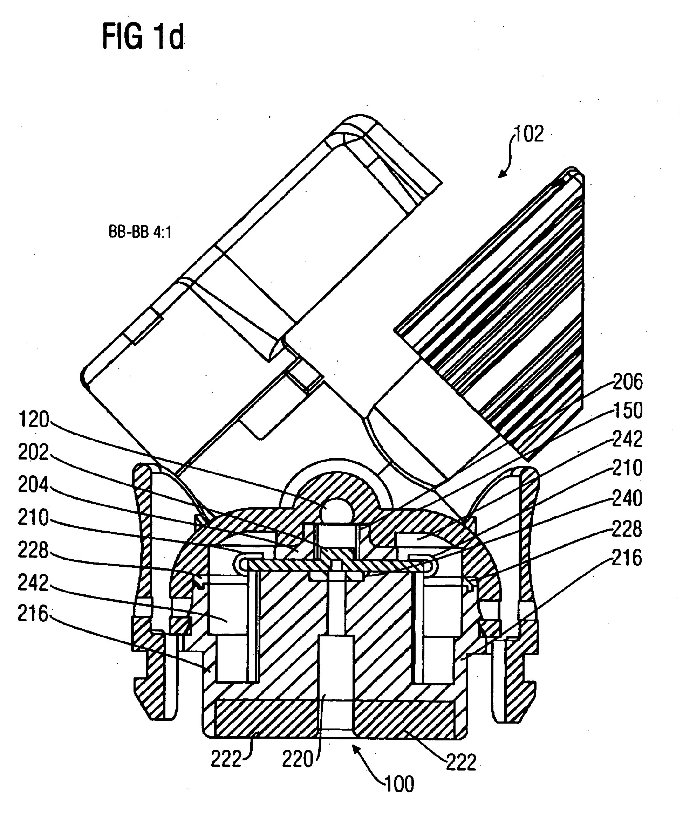

Die Fig. 1B zeigt die in Fig. 1A dargestellte Anordnung aus einem anderen Blickwinkel. Hierbei sind jedoch zur Erläuterung der nachfolgenden Figuren 1C und 1D bereits zwei Schnittlinien durch den Transducer 100, nämlich die erste Schnittlinie BA-BA und die zweite Schnittlinie BB-BB eingetragen. Wie nachfolgend noch detaillierter dargestellt wird, verläuft der Schnitt entlang der Schnittlinie BA-BA durch zwei buchsenförmige Anschlusskontakte. Diese Anschlusskontakte (welche in Fig. 1B nicht sichtbar sind) bilden die Anschlusskontakte einer Wheatstone-Messbrücke, wobei jedoch die beiden entlang der Schnittlinie BA-BA nachfolgend näher dargestellten Kontakte lediglich zwei Kontakte der Wheatstone-Messbrücke darstellen. Zwei weitere Kontakte der Wheatstone-Messbrücke sind diagonal zu den entlang der Schnittlinie BA-BA vorhandenen Anschlusskontakten angeordnet, es sind also vier Kontakte einer Wheatstone-Messbrücke in Bereichen 110 angeordnet, was jedoch aus Fig. 1B nicht direkt ersichtlich ist.

Ferner ist in Fig. 1B dargestellt, wie sich ein Durchflusskanal 120 im Bereich des Transducers 100 bzw. der Fluiddruckmesseinheit verengt, wie es dem Venturi-Prinzip entspricht.

Weiterhin kann auch, was jedoch in Fig. 1B nicht dargestellt ist, ein Sichtfenster in Form einer Vergrößerungslupe im verengten Teil des Durchflusskanals 120 angeordnet sein, so dass ein optisch vergrößert einsehbarer Bereich, vorzugsweise auf den Messchip, realisiert werden kann, wodurch dann durch einen Anwender des Transducers 100 überprüft werden kann, ob sich im Durchflusskanal Bläschen über dem Messchip abgesetzt haben.

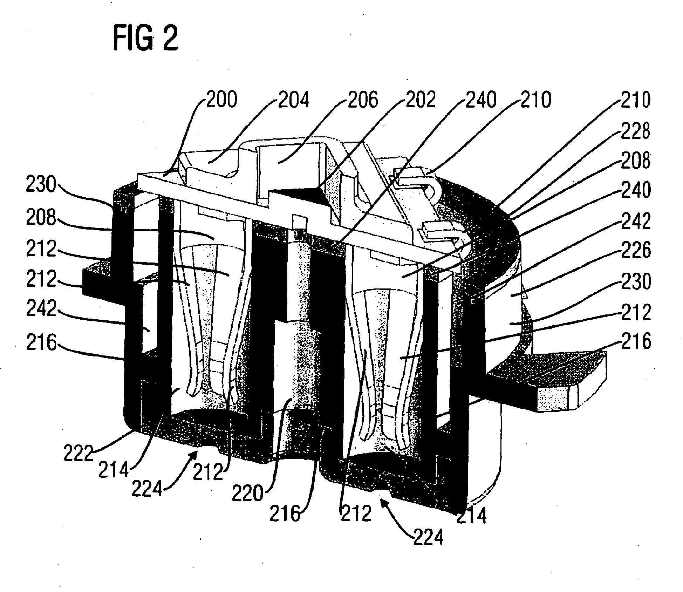

Aus den Figuren 1C und 1D ist ersichtlich, dass der Transducer ein oberes Teilelement 150 (Fluidkanal-Anschlusselement) aufweist, das einen Teil des Durchflusskanals 120 sowie einen oberen Gehäusedeckel des Transducers 100 bildet. Ferner ist aus den Figuren 1C und 1D ersichtlich, dass sich auch der Innenaufbau des Transducers 100 aus einer Vielzahl von Komponenten zusammensetzt, die aus darstellungstechnischen Gründen anhand der Fig. 2 näher beschrieben werden sollen.

Die Fig. 2 zeigt eine perspektivische Querschnittsdarstellung eines Teils des Transducers 100, wie er in Fig. 1C dargestellt ist. Insbesondere ist in Fig. 2 das zusammengesetzte bzw. fertighergestellte untere Teilelement des Transducers 100 gezeigt. Aus Gründen der Übersichtlichkeit wird im Folgenden zunächst der Aufbau des in Fig. 2 dargestellten unteren Teilelements, d.h. Gehäuseelement erläutert, woran anschließend dessen Herstellung anhand eines Ausführungsbeispiels erläutert wird.

Das in Fig. 2 dargestellte untere Element des Transducers 100 weist im fertig hergestellten Zustand einen Chipträger 200 auf, an dessen erster Seite ein Sensor 202 angeordnet bzw. befestigt ist. Der Chipträger 200 kann eine Hybridkeramik mit aufgedruckten Leiterbahnen und Widerständen sein. Für den Sensor 202 kann beispielsweise eine integrierte Schaltung (Chip) verwendet werden. Um den Sensor 202 zu schützen, ist ebenfalls an der ersten Seite des Chipträgers 200 ein Sensordom 204 angeordnet, so dass eine von dem Chipträger 200 vorstehende Wand des Sensordoms 204 den Sensor 202 umgibt. Nicht eingezeichnet ist eine Gelfüllung, die beispielsweise in eine durch den Sensordom 204 und den Chipträger 200 gebildete Ausnehmung 206 eingefüllt wird, in der sich der Sensor 202 befindet, um den Sensor 202 abzudecken. Ferner weist das in Fig. 2 dargestellte untere Teilelement Buchsenkontakte 208 mit Lötfahnen 210 und Kontaktfedern 212 auf. Die Lötfahnen können sich entweder auf die erste Seite des Chipträgers erstrecken oder sich nur auf die zweite Seite des Chipträgers erstrecken. In Fig. 2 ist dabei nur die erstgenannte Möglichkeit dargestellt. Der Sensordom ist vorzugsweise mit einem Gel gefüllt, das den Sensor 202 fluiddicht vom Fluidkanal abdichtet, jedoch eine Druckübertragung ermöglicht. Die Buchsenkontakte 208 sind dabei auf einer der ersten Seite des Chipträgers 200 gegenüberliegenden zweiten Seite an diesem angebracht. Weiterhin sind die Buchsenkontakte in jeweils einer Öffnung 214 des Gehäuseunterteils 216 angeordnet. Die Öffnung 214 des Gehäuseunterteils 216 erstreckt sich von einer ersten Seite des Gehäuseunterteils 216 aus bis zu einer der ersten Seite des Gehäuseunterteils 216 gegenüberliegenden zweiten Seite. In dem Gehäuseunterteil 216 ist ferner eine Mittelöffnung 220 angeordnet, die als Belüftungsloch für den Sensorchip 202 dient. Zur Abdichtung des Belüftungsloches 220 ist am Gehäuseunterteil 216 ein Dichtelement 240 angespritzt. Weiterhin sind die Öffnungen 214 in dem Gehäuseunterteil 216 an der zweiten Seite des Kontaktträgers durch eine angespritzte Dichtmembran 222 verschlossen, die beispielsweise aus einem TPE (TPE = Thermoplastisches Elastomer) sein kann. Ferner weist die angespritzte Dichtmembran 222 im Mittelbereich ein Sackloch 224 auf. Bei einem Sackloch kann der geschlossene Bereich durchgestanzt werden, um einen Durchgang zu ermöglichen. Alternativ kann auch ein Durchgangsloch in der angespritzten Dichtmembran 222 ausgebildet sein, was jedoch in Fig. 2 nicht dargestellt ist.

Ferner kann das Gehäuseunterteil 216 an einem Übergang 226 eine angespritzte Dichtung 228 aufweisen, die als Dichtlippe bei einem Zusammenfügen bzw. Verbinden des in Fig. 2 dargestellten unteren Teilelementes mit dem in Fig. 1C bzw. 1D dargestellten oberen Teilelement 150 des Transducers dient. Die Dichtlippe 228 kann dabei vorzugsweise ebenfalls aus TPE bestehen, so dass das Gehäuseunterteil einstückig mit allen erforderlichen Dichtelementen hergestellt werden kann. Auch das als Ersatz des O-Ringes wirkende Dichtelement 240 kann dabei aus einem TPE-Material hergestellt sein.

Zur Herstellung der Komponente der Fluiddruckmesseinheit wird nun zunächst der Chipträger 200 mit dem darauf angeordneten Messchip 202 sowie den Buchsenkontakten 208 mit den Lötfahnen 210 bereitgestellt. Die Lötfahnen werden an Kontaktanschlüsse auf dem Chipträger 200 angelötet, wobei die Kontaktanschlüsse eine Kontaktierung des auf dem Chipträger 200 angeordneten Chips 202 ermöglichen bzw. mit entsprechenden Chip-Pads elektrisch leitfähig verbunden sind. Das Verlöten wird dabei unter Umbiegen oder mit bereits vorgebogenen Lötfahnen 210 derart ausgeführt, dass die Buchsenkontakte 208 an einer zweiten Seite des Chipträgers von diesem hervorstehen, während der Chip 202 und die Kontaktanschlüsse auf der gegenüberliegenden ersten Seite des Chipträgers 200 angeordnet sind. Alternativ kann der Chipträger jedoch auch mit Leitungsbahnen bzw. Durchkontaktierungen derart versehen sein, dass die Kontaktanschlüsse 210 auf der zweiten Seite des Chipträger angeordnet sind.

Weiterhin kann auf derjenigen Seite des Chipträgers 200, an der der Sensor 202 angeordnet ist, der Sensordom 204 befestigt (beispielsweise aufgeklebt) werden. Schließlich erfolgt vorzugsweise ein Verfüllen des Sensordoms mit einem Gel, derart, dass das Gel den Messchip 202 umgibt (in Fig. 2 ist das Gel in dem Sensordom aus Darstellungsgründen jedoch nicht wiedergegeben). Durch das Gel kann nunmehr der Messchip 202 fluiddicht "verpackt" werden, so dass keine leitfähige Flüssigkeit, wie beispielsweise die Infusionslösung in direktem Kontakt mit dem Messchip bzw. mit Leiterbahnen auf dem Chipträger 200 in Berührung kommt und somit zu einer Verfälschung der Messwerte führt. Bis hierhin ist ein Messelement, bestehend aus dem Chipträger 200, dem Sensor 202, den Buchsenkontakten 208 sowie dem Sensordom 204 und der Gelfüllung hergestellt worden.

In einem nachfolgenden oder auch parallelen Schritt kann an das untere Teilelement 216 in einem Verbindungsbereich die Dichtlippe 228, eine Dichtmembran 222 und/oder eine Dichtung 240 angespritzt werden. Viele verschiedene Varianten für ein derartiges Anspritzen stehen zur Auswahl, welche insbesondere auch kostengünstig eingesetzt werden können.

Das wie oben beschrieben erzeugte Messelement bestehend aus dem Chipträger 200, dem Sensor 202, den Buchsenkontakten 208 sowie dem Sensordom 204 mit der Füllung des Sensordomes wird dann mit dem unteren Teilelement 216 zusammengefügt, beispielsweise eingepresst. Hierbei können die Buchsenkontakte 208 direkt in die Öffnungen 214 eingeschoben werden, wodurch sich der in der Figur 2 dargestellte Aufbau ergibt. Alternativ kann jedoch auch ein loses Einlegen des Messelementes in das Gehäuseelement erfolgen, sodass die Buchsenkontakte in den Öffnungen 214 etwas Spiel haben, wobei ein Anpressen und somit ein Abdichten des Dichtelementes 240 an den Chipträger 200 durch ein nachfolgendes Zusammenpressen des Gehäuseelementes 216 an das Fluidkanal-Anschlusselement 150, das in Abb. 2 nicht eingezeichnet ist, erfolgt. Hierdurch wird die Komponente der Fluiddruckmesseinheit hergestellt.

Die so hergestellte Komponente der Fluiddruckmesseinheit ist dazu vorgesehen, an das obere Teilelement 150 (d.h. das Fluidkanal-Anschlusselement) angefügt zu werden, so dass der Sensor 202 über die Gelfüllung fluidisch mit dem Durchflusskanal 120 verbunden ist und Druckschwankungen eines Fluids in dem Durchflusskanal 120 über die Gelfüllung auf den Messchip 202 übertragen werden. Die Klebe- bzw. Haftfläche zwischen dem Messelement und dem oberen Teilelement 150 kann dabei beispielsweise zwischen Sensordom 204 und dem oberen Teilelement 150 liegen und eine Abdichtung eines Zwischenraumes 242 in dem unteren Teilelement sicherstellen.

Das Verbinden des oberen Teilelementes 150 mit dem unteren Teilelement 216 kann beispielsweise durch ein Verkleben erfolgen, derart, dass eine in Fig. 2 dargestellte äußere Seitenwand 230 mit Klebematerial versehen wird und mit dem in Fig. 1C und 1D dargestellten Transducer-Deckel bzw. - Kanalaufsatz 150 zusammengefügt wird. Alternativ kann jedoch auch ein Schnappverschluss oder ein Schraubverschluss verwendet werden, bei dem durch ein Zusammendrücken des oberen und unteren Teilelementes und das damit verbundene Zusammenpressen der Dichtlippe 228 eine fluiddichte Verbindung zwischen diesen beiden Teilelementen und eine Abdichtung des Innenraums 242 entsteht. Zu beachten ist jedoch, dass bei einem Schraubverschluss der Sensordom 204 nicht wie in Figur 2 dargestellt, rechteckig, sondern kreisförmig ausgebildet sein sollte. Eine derartige Kombinationsmöglichkeit entweder durch Kleben oder durch eine mechanische Verbindung, wie einen Schnapp- oder Schraubverschluss verbunden mit der Tatsache, dass das Gehäuseunterteil 216 vorzugsweise bereits mit allen Dichtungen versehen wurde, bietet somit eine sehr einfache und kostengünstige Möglichkeit, den Transducer 100 herzustellen.

Das vorstehend erläuterte Ausführungsbeispiel des erfindungemäßen Verfahrens bieten den Vorteil, dass nicht mehr die Buchsenkontakte in den Kontaktträger eingepresst werden müssen, dann ein O-Ring auf den Kontaktträger gelegt und anschließend der Chipträger in Form der Hybridkeramik auf die Lötfahnen aufgesetzt und verlötet wird, was eine hohe thermische oder stabilitätsbelastende Auswirkung auf den Kontaktträger hat. Auch kann ein Kostenvorteil durch die einstückige Fertigung des Gehäuseunterteils 216 mit allen erforderlichen Dichtungen im Zweikomponentenprozess realisiert werden.

Dabei kann ein dem O-Ring vergleichbares Dichtelement 240 zwischen Kontaktträger bzw. Gehäuseunterteil 216 einerseits, und dem Chipträger 200 andererseits realisiert werden, wie es beispielsweise mit den in Fig. 2 dargestellten Dichtelement 240 möglich ist. Dieses Dichtelement 240 kann zur Abdichtung der Buchsenkontakte 208 gegenüber dem Innenraum 242 dienen. Weiterhin kann auch das Dichtelement 240 als Abdichtung zwischen dem durch das Mittelloch 220 gebildeten Hohlraum und dem Chipträger 200 dienen, so dass der Innenraum 242 und die Buchsenkontakte 208 in den Bohrungen 214 gegeneinander und gegenüber dem Mittelloch 220 abgedichtet sind. Hierdurch wird sichergestellt, dass auch beispielsweise bei einer Undichtheit des Infusionssystems mit Austritt von Infusionslösung keine solche leitfähige Infusionslösung über das Mittelloch 220 and die buchsenförmigen Anschlusskontakte 208 gelangen kann und beispielsweise über "Kriechströme" eine Verfälschung des gemessenen Signals bewirkt. Zugleich kann im Bereich des Mittelloches 220 ferner eine Öffnung im Chipträger 200 vorgesehen sein, so dass ein Umgebungsluftdruck der Umgebungsluft unmittelbar an eine Rückseite des Messsensors gelangen kann, wodurch eine Kalibrierung des Messsensors 202 in verschiedenen Einsatzumgebungen ermöglicht wird, da die druckempfindliche Membran des Sensors (nicht dargestellt) zu beiden Seiten hin offen bzw. freiliegend ist, so dass bei beidseitig anliegendem Athmosphärendruck keine Vorspannung der Membran existiert.

Bezüglich der Dichtmembran 222, welche an der zweiten Seite des Gehäuseunterteil 216 die Öffnungen 214 verschließt, sei angemerkt, dass die Kontaktierung entweder mittels eines Durchstoßens der Kontaktelektrode durch das Sackloch 224 erfolgen kann oder, wenn das Loch in Form eines Durchgangslochs ausgebildet ist, die Kontaktierung mittels eines Hindurchsteckens des Kontaktes durch dieses Durchgangsloch erfolgen kann.

Das Ausbilden der dichten Membran 222 mit den Sacklöchern 224, welche zu durchstoßen ist bzw. das Ausbilden der Durchgangslöcher in der Dichtmembran 222 und das nachfolgende Hindurchstecken der Kontaktelektrode bringt somit den Vorteil, dass, insbesondere bei der Ausbildung von kreisförmigen Sack- bzw. Durchgangslöchern, die üblicherweise kreisförmige Kontaktelektrode durch ein "Abstreifen" weitestgehend von Verschmutzungen wie beispielsweise der leitfähigen Infusionsflüssigkeit, gereinigt werden kann, so dass die Wahrscheinlichkeit für das Einbringen von elektrisch leitfähigen Verschmutzungen an die buchsenförmigen Anschlusskontakte 208 mit den Kontaktfedern 212 deutlich reduziert wird.

Zusammenfassend geht aus obigen Ausführungsbeispielen ein Ansatz hervor, bei dem die Buchsenkontakte direkt an die Keramik bzw. den Chipträger angelötet werden, woraufhin die Keramik beispielsweise mit dem Gehäuseoberteil verklebt oder zusammengesetzt wird. Ein Einpressen der Buchsenkontakte in das Gehäuseunterteil ist nicht mehr notwendig. Vielmehr haben die Buchsenkontakte im Gehäuseunterteil etwas Spiel. Da der Lötprozess nicht mehr in Verbindung mit dem Kontaktträger oder dem Gehäuseunterteil steht, kann das Gehäuseunterteil einstückig gefertigt werden. Die Werkstoffe können beispielsweise so gewählt werden, dass das Gehäuseunterteil als Zweikomponententeil gefertigt werden kann (beispielsweise eine spezielle Kombination des Materials des Gehäuseunterteils (wie z.B. Polypropylen) und einem Material zum Anspritzen der Dichtungen (wie beispielsweise TPE). Hierbei sind dann das Gehäuseunterteil, der Kontaktträger, die Dichtmembrane und der O-Ring sowie die Dichtlippe vorzugsweise als ein einziges Teil ausgebildet. Darüber hinaus ist eine Ultraschallverschweißung nicht mehr erforderlich, da die Dichtheit zwischen Gehäuseober- und Unterteil vorzugsweise durch eine ebenfalls angespritzte Dichtlippe erzielt werden kann.

In anderen Worten ausgedrückt besteht die Erfindung in einem Verfahren zum Herstellen einer Komponente einer Fluiddruckmesseinheit (100) mit einem Fluidkanal-Anschlusselement (150), das ein Fluiddruckmessvolumen (120) mit einer Anschlussöffnung aufweist, mit den Schritten: Bereitstellen eines Messelementes mit einem auf einer ersten Seite eines Chipträges (200) angeordneten Messchip (202) und einer Mehrzahl von buchsenförmigen Anschlusskontakten (208) zur Kontaktierung von Kontaktanschlüssen des Messchips (202), wobei die buchsenförmigen Anschlusskontakte (208) von einer der ersten Seite des Chipträgers (200) gegenüberliegenden zweiten Seite des Chipträgers (200) hervorstehen; Bereitstellen eines Gehäuseelementes (216), das eine Mehrzahl von Aufnahmelöchern (214) in einer ersten Seite des Gehäuseelementes aufweist, die ausgebildet sind, um bei einem Zusammenfügen des Gehäuseelementes (216) mit dem Messelement die buchsenförmigen Anschlusskontakte (208) aufzunehmen, wobei sich die Aufnahmelöcher (214) von der ersten Seite des Gehäuseelementes (216) aus zu einer der ersten Seite des Gehäuseelementes (216) gegenüberliegenden zweiten Seite des Gehäuseelementes (216) hin erstrecken, wobei das Bereitstellen des Gehäuseelementes (216) ein Anspritzen eines Verschlusselementes (222) an die zweite Seite des Gehäuseelementes (216) zum Verschließen der Aufnahmelöcher (214) an der zweiten Seite des Gehäuseelementes (216) umfasst; und Zusammenfügen des Messelementes und des Gehäuseelementes (216) derart, dass die zweite Seite des Chipträgers (200) der ersten Seite des Gehäuseelementes (216) gegenüber liegt und die buchsenförmigen Anschlusskontakte (208) in den Aufnahmelöchern (214) angeordnet werden, um die Komponente der Fluiddruckmesseinheit (100) herzustellen, wobei die Anschlussöffnung ausgebildet ist, um das Messelement oder ein den Messchip umgebendes Gel mit dem Fluiddruckmessvolumen zu koppeln. Dabei kann der Schritt des Bereitstellens des Messelementes ein Verlöten von Lötanschlüssen (210) der buchsenförmigen Anschlusskontakte (208) mit den Kontaktanschlüssen des Messchips (202) umfassen. Alternativ oder zusätzlich kann der Schritt des Bereitstellens des Messelementes ein Anbringen eines Sensordomes (204) auf der ersten Seite des Chipträgers (200) umfassen, wobei der Sensordom (204) den Messchip (202) umgibt und wobei der Schritt des Bereitstellen ferner ein Befüllen des Sensordoms (204) mit einem Gel umfasst, derart, dass der Messchip (202) von dem Gel abgedeckt ist. Ferner alternativ oder zusätzlich kann das Bereitstellen des Gehäuseelementes (216) ferner das Bilden von Kontaktierungslöchern (224) in dem Verschlusselement im Bereich der Aufnahmelöcher (214) umfassen. Schließlich wird auch darauf hingewiesen, dass der Schritt des Bereitstellen des Gehäuseelementes (216) auch ein Ausbilden eines Basiskörpers (216) mit den Aufnahmelöchern (214) aus einem thermoplastischem Material und ein Anspritzen von einem thermoplastischen Elastomer zum Ausbilden des ersten und zweiten Abdichtelementes (228, 240) und des Verschlusselementes (222) umfassen kann.

Auf ähnliche Weise besteht die vorliegende Erfindung in einer Komponente zum Einsatz in einer Fluiddruckmesseinheit (100), wobei die Komponente folgende Merkmale aufweist: ein Gehäuseelement (216) mit einer Mehrzahl von Aufnahmelöchern (214), die sich von einer ersten Seite des Gehäuseelementes (216) aus zu einer der ersten Seite des Gehäuseelementes gegenüberliegenden zweiten Seite des Gehäuseelementes (216) hin erstrecken, wobei das Gehäuseelement (216) auf der zweiten Seite ein Verschlusselement (222) zum Verschließen der Aufnahmeöffnungen (214) an der zweiten Seite des Gehäuseelementes (216) aufweist, das einstückig mit dem Gehäuseelement (216) verbunden ist, wobei sich das Material des Verschlusselementes (222) von einem Material des Gehäuseelementes (216) unterscheidet; und ein Messelement mit einem auf einer ersten Seite eines Chipträgers (200) angeordneten Messchip (202) und einer Mehrzahl von buchsenförmigen Anschlusskontakten (208) zur Kontaktierung von Kontaktanschlüssen des Messchips (202), wobei die buchsenförmigen Anschlusskontakte (208) von einer der ersten Seite des Chipträgers (200) gegenüberliegenden zweiten Seite des Chipträgers (200) hervorstehen, wobei das Messelement und das Gehäuseelement (216) derart aufgebaut sind, dass das Messelement und das Gehäuseelement (216) derart zusammenfügbar sind, dass in einem zusammengefügten Zustand die buchsenförmigen Anschlusskontakte (208) in die Aufnahmelöcher (214) hineinragen und die zweite Seite des Chipträgers (200) der ersten Seite des Gehäuseelementes (216) gegenüber liegt, um die Komponente zum Einsatz in der Fluiddruckmesseinheit (100) zu bilden.

Bei der Komponente kann das Messelement über Leiterbahnen zwischen Lötanschlüssen (210) der buchsenförmigen Anschlusskontakte (208) und den Kontaktanschlüssen des Messchips (202) eine Lotschicht dielektrisch verbunden sein. Ferner kann das Messelement einen Sensordom (204) auf der ersten Seite des Chipträgers (200) umfassen, wobei der Sensordom (204) den Messchip (202) umgibt und wobei der Sensordom (204) mit einem Gel befüllt ist, derart, dass der Messchip (202) von dem Gel abgedeckt ist.

## Patentansprüche

1. Verfahren zum Herstellen einer Komponente einer Fluiddruckmesseinheit (100) mit einem Fluidkanal-Anschlusselement (150), das ein Fluiddruckmessvolumen (120) mit einer Anschlussöffnung aufweist, mit folgenden Schritten:
Bereitstellen eines Messelementes mit einem auf einer ersten Seite eines Chipträges (200) angeordneten Messchip (202) und einer Mehrzahl von buchsenförmigen Anschlusskontakten (208) zur Kontaktierung von Kontaktanschlüssen des Messchips (202), wobei die buchsenförmigen Anschlusskontakte (208) von einer der ersten Seite des Chipträgers (200) gegenüberliegenden zweiten Seite des Chipträgers (200) hervorstehen;
Bereitstellen eines Gehäuseelementes (216), das eine Mehrzahl von Aufnahmelöchern (214) in einer ersten Seite des Gehäuseelementes aufweist, die ausgebildet sind, um bei einem Zusammenfügen des Gehäuseelementes (216) mit dem Messelement die buchsenförmigen Anschlusskontakte (208) aufzunehmen, wobei sich die Aufnahmelöcher (214) von der ersten Seite des Gehäuseelementes (216) aus zu einer der ersten Seite des Gehäuseelementes (216) gegenüberliegenden zweiten Seite des Gehäuseelementes (216) hin erstrecken,
wobei das Bereitstellen des Gehäuseelementes (216) ein Anspritzen eines Verschlusselementes (222) an die zweite Seite des Gehäuseelementes (216) zum Verschließen der Aufnahmelöcher (214) an der zweiten Seite des Gehäuseelementes (216) umfasst; und
Zusammenfügen des Messelementes und des Gehäuseelementes (216) derart, dass die zweite Seite des Chipträgers (200) der ersten Seite des Gehäuseelementes (216) gegenüber liegt und die buchsenförmigen Anschlusskontakte (208) in den Aufnahmelöchern (214) angeordnet werden, um die Komponente der Fluiddruckmesseinheit (100) herzustellen,
wobei die Anschlussöffnung ausgebildet ist, um das Messelement oder ein den Messchip umgebendes Gel mit dem Fluiddruckmessvolumen zu koppeln.

2. Verfahren gemäß Anspruch 1, bei dem das Bereitstellen des Gehäuseelementes (216) auch ein Anspritzen eines ersten Abdichtelementes (240) an die erste Seite des Gehäuseelementes (216) umfasst, wobei das Zusammenfügen des Messelementes und des Gehäuseelementes (216) derart durchgeführt wird, dass das erste Abdichtelement an die zweite Seite des Chipträgers (200) angepresst wird, um die Komponente der Fluiddruckmesseinheit (100) herzustellen.

3. Verfahren gemäß Anspruch 2, bei dem der Schritt des Bereitstellens des Gehäuseelementes (216) ein Bilden einer Mittelöffnung (220) zwischen den Aufnahmelöchern (214) umfasst, sich die Mittelöffnung (220) von der ersten Seite des Gehäuseelementes (216) zu der zweiten Seite des Gehäuseelementes (216) erstreckt, der Schritt des Bereitstellens des Messelementes ein Bereitstellen einer Öffnung in dem Chipträger (200) unter dem Messchip umfasst, das erste Abdichtelement (240) so ausgebildet und der Schritt des Zusammenfügens so durchgeführt wird, dass das Anpressen des ersten Abdichtelementes an die zweite Seite des Chipträgers (200) zu einer Abdichtung der Aufnahmelöcher (214) gegenüber sowohl der Mittelöffnung (220) als auch einem Innenraum (242) führt, der sich bei einer Verbindung des Fluidkanal-Anschlusselements (150) mit dem Gehäuseelement (216) zwischen dem Fluidkanal-Anschlusselement (150) und dem Gehäuseelement ergibt, und dass in dem zusammengefügten Zustand das Gehäuseelement (216) derart mit dem Fluidkanal-Anschlusselement (150) verbindbar ist, dass eine Druckübertragung von einem in dem Fluiddruckmessvolumen (120) befindlichen Fluid über die Anschlussöffnung auf den Messchip (202) ermöglicht wird.

4. Verfahren gemäß Anspruch 2 oder 3, bei dem der Schritt des Anspritzens ferner ein Anspritzen eines zweiten Abdichtelementes (228) an die erste Seite des Gehäuseelementes (216) umfasst, und bei dem das Zusammenfügen des Messelementes und des Gehäuseelementes (216) ferner derart durchgeführt wird, dass in dem zusammengefügten Zustand das Gehäuseelement (216) derart mit dem Fluidkanal-Anschlusselement (150) verbindbar ist, dass eine Druckübertragung von einem in dem Fluiddruckmessvolumen (120) befindlichen Fluid über die Anschlussöffnung auf den Messchip (202) ermöglicht wird und das zweite Abdichtelement (228) durch ein Zusammendrücken des Fluidkanal-Anschlusselements (150) und des Gehäuseelements (216) zu einer fluiddichten Verbindung zwischen denselben führt.

5. Verfahren gemäß einem der Ansprüche 1 bis 4, bei dem der Schritt des Bereitstellens des Messelementes ein elektrisches Verbinden von Lötanschlüssen (210) der buchsenförmigen Anschlusskontakte (208) mit den Kontaktanschlüssen des Messchips (202) mittels Löten umfasst und das Zusammenfügen des Messelementes und des Gehäuseelementes (216) erst im Anschluss an das elektrische Verbinden stattfindet.

6. Verfahren zum Herstellen einer Fluiddruckmesseinheit mit folgenden Schritten:
Durchführen eines Verfahren gemäß einem der vorhergehenden Ansprüche;
Bereitstellen des Fluidkanal-Anschlusselementes (150), das ein Fluiddruckmessvolumen (120) mit einer Anschlussöffnung zur fluidischen Kopplung mit dem Fluiddruckmessvolumen (120) aufweist; und
Verbinden der Komponente der Fluiddruckmesseinheit mit dem Fluidkanal-Anschlusselement (150) derart, dass eine Druckübertragung von einem in dem Fluiddruckmessvolumen (120) befindlichen Fluid auf den Messchip (202) durch die Anschlussöffnung ermöglicht wird.

7. Verfahren gemäß Anspruch 6, bei dem der Schritt des Verbindens der Komponente der Fluiddruckmesseinheit ein Andrücken des Messelementes an das Gehäuseelement (216) umfasst.

8. Verfahren gemäß Anspruch 6 oder 7, bei dem das Bereitstellen des Fluidkanal-Anschlusselementes (150) ein Bereitstellen eines Fluidkanals (120) umfasst, der an einer Stelle im Bereich der Anschlussöffnung einen geringeren Querschnitt aufweist, als an einer weiteren Stelle, und wobei im Bereich der Anschlussöffnung ein Sichtfenster in einer Wand des Fluidkanals ausgebildet ist, derart, dass eine Einsichtnahme auf den Messchip (202) oder ein den Messchip (202) abdeckendes Gel von außerhalb der Fluiddruckmesseinheit (100) ermöglicht wird.

9. Verfahren gemäß einem der Ansprüche 6 bis 8, mit einem Verfahren, bei dem der Schritt des Bereitstellens des Messelementes ein Anbringen eines Sensordomes (204) auf der ersten Seite des Chipträgers (200) umfasst, wobei der Sensordom (204) den Messchip (202) umgibt und wobei der Schritt des Bereitstellen ferner ein Befüllen des Sensordoms (204) mit einem Gel umfasst, derart, dass der Messchip (202) von dem Gel abgedeckt ist, wobei das Befüllen vor dem Verbinden stattfindet.

10. Komponente zum Einsatz in einer Fluiddruckmesseinheit (100), wobei die Komponente folgende Merkmale aufweist:
ein Gehäuseelement (216) mit einer Mehrzahl von Aufnahmelöchern (214), die sich von einer ersten Seite des Gehäuseelementes (216) aus zu einer der ersten Seite des Gehäuseelementes gegenüberliegenden zweiten Seite des Gehäuseelementes (216) hin erstrecken, wobei das Gehäuseelement (216) auf der zweiten Seite ein Verschlusselement (222) zum Verschließen der Aufnahmeöffnungen (214) an der zweiten Seite des Gehäuseelementes (216) aufweist, das einstückig mit dem Gehäuseelement (216) verbunden ist, wobei sich das Material des Verschlusselementes (222) von einem Material des Gehäuseelementes (216) unterscheidet; und
ein Messelement mit einem auf einer ersten Seite eines Chipträgers (200) angeordneten Messchip (202) und einer Mehrzahl von buchsenförmigen Anschlusskontakten (208) zur Kontaktierung von Kontaktanschlüssen des Messchips (202), wobei die buchsenförmigen Anschlusskontakte (208) von einer der ersten Seite des Chipträgers (200) gegenüberliegenden zweiten Seite des Chipträgers (200) hervorstehen,
wobei das Messelement und das Gehäuseelement (216) derart aufgebaut sind, dass das Messelement und das Gehäuseelement (216) derart zusammenfügbar sind, dass in einem zusammengefügten Zustand die buchsenförmigen Anschlusskontakte (208) in die Aufnahmelöcher (214) hineinragen und die zweite Seite des Chipträgers (200) der ersten Seite des Gehäuseelementes (216) gegenüber liegt, um die Komponente zum Einsatz in der Fluiddruckmesseinheit (100) zu bilden.

11. Komponente gemäß Anspruch 10, bei dem das Gehäuseelement (216) an der ersten Seite ein erstes Abdichtelement (240) aufweist, das einstückig mit dem Gehäuseelement (216) verbunden ist, wobei sich ein Material des ersten Abdichtelementes (240) von einem Material des Gehäuseelementes (216) unterscheidet, und bei dem das Messelement und das Gehäuseelement (216) derart zusammenfügbar sind, dass in dem zusammengefügten Zustand das erste Abdichtelement an die zweite Seite des Chipträgers (200) angepresst wird.

12. Vorrichtung gemäß Anspruch 11, bei der das Gehäuseelementes (216) eine Mittelöffnung (220) zwischen den Aufnahmelöchern (214) umfasst, die sich von der ersten Seite des Gehäuseelementes (216) zu der zweiten Seite des Gehäuseelementes (216) erstreckt, das Messelement eine Öffnung in dem Chipträger (200) unter dem Messchip umfasst, das erste Abdichtelement (240) so ausgebildet ist und das Messelement und das Gehäuseelement (216) derart zusammenfügbar sind, dass das Anpressen des ersten Abdichtelementes an die zweite Seite des Chipträgers (200) zu einer Abdichtung der Aufnahmelöcher (214) gegenüber sowohl der Mittelöffnung (220) als auch einem Innenraum (242) führt, der sich bei einer Verbindung des Fluidkanal-Anschlusselements (150) mit dem Gehäuseelement (216) zwischen dem Fluidkanal-Anschlusselement (150) und dem Gehäuseelement ergibt, und dass in dem zusammengefügten Zustand das Gehäuseelement (216) derart mit dem Fluidkanal-Anschlusselement (150) verbindbar ist, dass eine Druckübertragung von einem in dem Fluiddruckmessvolumen (120) befindlichen Fluid über die Anschlussöffnung auf den Messchip (202) ermöglicht wird.

13. Komponente gemäß Anspruch 12, bei dem das Messelement und das Gehäuseelement (216) zudem derart aufgebaut sind, dass das Messelement und das Gehäuseelement (216) derart zusammenfügbar sind, dass in dem zusammengefügten Zustand das Gehäuseelement (216) derart mit dem Fluidkanal-Anschlusselement (150) verbindbar ist, dass eine Druckübertragung von einem in dem Fluiddruckmessvolumen (120) befindlichen Fluid über die Anschlussöffnung auf den Messchip (202) ermöglicht wird und das zweite Abdichtelement (228) durch ein Zusammendrücken des Fluidkanal-Anschlusselements (150) und des Gehäuseelements (216) zu einer fluiddichten Verbindung zwischen denselben führt.

14. Komponente gemäß einem der Ansprüche 11 bis 13, bei dem das Gehäuseelement (216) eine Mittelöffnung (220) zwischen den Aufnahmelöchern (214) umfasst, wobei sich die Mittelöffnung (220) von der ersten Seite des Gehäuseelementes (216) zu der zweiten Seite des Gehäuseelementes (216) erstreckt, wobei eine Öffnung in dem Chipträger (200) unter dem Messchip vorgesehen ist, die Mittelöffnung (220) mit der Öffnung des Chipträgers in Verbindung ist, der Messchip (202) auf dessen Rückseite über die Öffnung des Chipträgers und die Mittelöffnung mit einer Außenumgebung der Komponente der Fluiddruckmesseinheit (100) verbunden ist und in dem zusammengefügten Zustand die Buchsenkontakte durch das erste Abdichtelement (240) gegenüber der Mittelöffnung und einem Zwischenraum (242) zwischen Gehäuseelement (216) und dem Fluidkanal-Anschlusselement (150), der sich bei einer Verbindung des Fluidkanal-Anschlusselements (150) mit dem Gehäuseelement (216) ergibt, abgedichtet sind.

15. Komponente gemäß einem der Ansprüche 10 bis 14, bei dem das Gehäuseelement (216) ferner eine Mehrzahl von Kontaktierungslöchern (224) in dem Verschlusselement im Bereich der Aufnahmelöcher (214) aufweist.

16. Fluiddruckmesseinheit mit
einer Komponente gemäß einem der Ansprüche 10 bis 15; und
dem Fluidkanal-Anschlusselement (150), das ein Fluiddruckmessvolumen (120) mit einer Anschlussöffnung zur fluidischen Kopplung mit dem Fluiddruckmessvolumen aufweist,
wobei das Gehäuseelement der Komponente über einen Schnapp- oder Schraubverschluss mit dem Fluidkanal-Anschlusselement (150) verbindbar ist.

## Claims

1. A method for manufacturing a component of a fluid pressure measurement unit (100) comprising a fluid channel terminal element (150) having a fluid pressure measurement volume (120) with a terminal opening, comprising:
providing a measurement element with a measurement chip (202) arranged on a first side of a chip carrier (200) and a plurality of socket-shaped terminal contacts (208) for contacting contact terminals of the measurement chip (202), wherein the socket-shaped terminal contacts (208) protrude from a second side of the chip carrier (200) opposite from the first side of the chip carrier (200);
providing a housing element (216) comprising a plurality of receiving holes (214) in a first side of the housing element which are implemented to receive the socket-shaped terminal contacts (208) when combining the housing element (216) with the measurement element, wherein the receiving holes (214) extend from the first side of the housing element (216) to a second side of the housing element (216) opposite from the first side of the housing element (216),
wherein providing the housing element (216) includes moulding a sealing element (222) to the second side of the housing element (216) for closing the receiving holes (214) on the second side of the housing element (216); and
combining the measurement element and the housing element (216) such that the second side of the chip carrier (200) is opposite from the first side of the housing element (216) and the socket-shaped terminal contacts (208) are arranged in the receiving holes (214) to manufacture the component of the fluid pressure measurement unit (100),
wherein the terminal opening is implemented to couple the measurement element or a gel surrounding the measurement chip to the fluid pressure measurement volume.

2. The method according to claim 1, wherein providing the housing element (216) includes moulding a first sealing element (240) to the first side of the housing element (216), wherein combining the measurement element and the housing element (216) is performed such that the first sealing element is pressed to the second side of the chip carrier (200) to manufacture the component of the fluid pressure measurement unit (100).

3. The method according to claim 2, wherein the step of providing the housing element (216) includes forming a central opening (220) between the receiving holes (214), the central opening (220) extends from the first side of the housing element (216) to the second side of the housing element (216), the step of providing the measurement element includes providing an opening in the chip carrier (200) below the measurement chip, the first sealing element (240) is implemented and the step of combining is performed such that pressing the first sealing element to the second side of the chip carrier (200) results in sealing the receiving holes (214) with respect to both the central opening (220) and an interior (242) forming between the fluid channel terminal element (150) and the housing element when connecting the fluid channel terminal element (150) to the housing element (216), and such that, in the combined state, the housing element (216) is connectable to the fluid channel terminal element (150) such that pressure transmission from a fluid located in the fluid pressure measurement volume (120) to the measurement chip (202) via the terminal opening is enabled.

4. The method according to claims 2 or 3, wherein the step of moulding further comprises moulding a second sealing element (228) to the first side of the housing element (216), and wherein combining the measurement element and the housing element (216) is further performed such that, in the combined state, the housing element (216) is connectable to the fluid channel terminal element (150) such that pressure transmission from a fluid located in the fluid pressure measurement volume (120) to the measurement chip (202) via the terminal opening is enabled and the second sealing element (228), by pressing together the fluid channel terminal element (150) and the housing element (216), results in a fluid-tight connection therebetween.

5. The method according to one of claims 1 to 4, wherein the step of providing the measurement element includes electrically connecting soldering terminals (210) of the socket-shaped terminal contacts (208) to the contact terminals of the measurement chip (202) by soldering, and combining the measurement element and the housing element (216) only takes place after electrically connecting.

6. A method for manufacturing a fluid pressure measurement unit, comprising:
performing a method according to one of the preceding claims;
providing a fluid channel terminal element (150) comprising a fluid pressure measurement volume (120) with a terminal opening for fluidic coupling to the fluid pressure measurement volume (120); and
connecting the component of the fluid pressure measurement unit to the fluid channel terminal element (150) such that pressure transmission from a fluid located in the fluid pressure measurement volume (120) to the measurement chip (202) by the terminal opening is enabled.

7. The method according to claim 6, wherein the step of connecting the component of the fluid pressure measurement unit includes pressing the measurement element to the housing element (216).

8. The method according to claims 6 or 7, wherein providing the fluid channel terminal element (150) includes providing a fluid channel (120) comprising a smaller cross-section at a location in the region of the terminal opening than at a further location, and wherein in the region of the terminal opening a viewing window is implemented in a wall of the fluid channel such that a view on the measurement chip (202) or a gel covering the measurement chip (202) from outside the fluid pressure measurement unit (100) is enabled.

9. The method according to one of claims 6 to 8, comprising a method in which the step of providing the measurement element includes attaching a sensor dome (204) to the first side of the chip carrier (200), wherein the sensor dome (204) surrounds the measurement chip (202), and wherein the step of providing further includes filling the sensor dome (204) with a gel such that the measurement chip (202) is covered by the gel, filling taking place before connecting.

10. A component for being used in a fluid pressure measurement unit (100), the component comprising:
a housing element (216) comprising a plurality of receiving holes (214) extending from a first side of the housing element (216) to a second side of the housing element (216) opposite from the first side of the housing element, wherein the housing element (216) comprises a closing element (222) on the second side for closing the receiving openings (214) on the second side of the housing element (216) which is integrally connected to the housing element (216), wherein the material of the closing element (222) is different from a material of the housing element (216); and
a measurement element with a measurement chip (202) arranged on a first side of a chip carrier (200) and a plurality of socket-shaped terminal contacts (208) for contacting contact terminals of the measurement chip (202), wherein the socket-shaped terminal contacts (208) protrude from a second side of the chip carrier (200) opposite from the first side of the chip carrier (200),
wherein the measurement element and the housing element (216) are constructed such that the measurement element and the housing element (216) may be combined such that, in a combined state, the socket-shaped terminal contacts (208) protrude into the receiving holes (214) and the second side of the chip carrier (200) is opposite from the first side of the housing element (216), in order to form the component for being used in the fluid pressure measurement unit (100).

11. The component according to claim 10, wherein the housing element (216) comprises a first sealing element (240) on the first side which is integrally connected to the housing element (216), wherein a material of the first sealing element (240) is different from a material of the housing element (216), and wherein the measurement element and the housing element (216) may be combined such that, in the combined state, the first sealing element is pressed to the second side of the chip carrier (200).

12. The device according to claim 11, wherein the housing element (216) includes a central opening (220) between the receiving holes (214) which extends from the first side of the housing element (216) to the second side of the housing element (216), the measurement element includes an opening in the chip carrier (200) below the measurement chip, the first sealing element (240) is implemented and the measurement element and the housing element (216) may be combined such that pressing the first sealing element to the second side of the chip carrier (200) results in sealing the receiving holes (214) with respect to both the central opening (220) and an interior (242) forming between the fluid channel terminal element (150) and the housing element when connecting the fluid channel terminal element (150) to the housing element (216), and such that, in the combined state, the housing element (216) is connectable to the fluid channel terminal element (150) such that pressure transmission from a fluid located in the fluid pressure measurement volume (120) to the measurement chip (202) via the terminal opening is enabled.

13. The component according to claim 12, wherein the measurement element and the housing element (216) are further constructed such that the measurement element and the housing element (216) may be combined such that, in the combined state, the housing element (216) is connectable to the fluid channel terminal element (150) such that pressure transmission from a fluid located in the fluid pressure measurement volume (120) to the measurement chip (202) via the terminal opening is enabled and the second sealing element (228), by pressing together the fluid channel terminal element (150) and the housing element (216), results in a fluid-tight connection therebetween.

14. The component according to one of claims 11 to 13, wherein the housing element (216) includes a central opening (220) between the receiving holes (214), wherein the central opening (220) extends from the first side of the housing element (216) to the second side of the housing element (216), wherein an opening is provided in the chip carrier (200) below the measurement chip, the central opening (220) is in connection with the opening of the chip carrier, the measurement chip (202) is connected on its back side to an exterior environment of the component of the fluid pressure measurement unit (100) via the opening of the chip carrier and the central opening and the socket contacts, in the combined state, are sealed by the first sealing element (240) with respect to the central opening and a gap (242) between the housing element (216) and the fluid channel terminal element (150) forming when connecting the fluid channel terminal element (150) to the housing element (216).

15. The component according to one of claims 10 to 14, wherein the housing element (216) further comprises a plurality of contacting holes (224) in the closing element in the area of the receiving holes (214).

16. A fluid pressure measurement unit comprising:
a component according to one of claims 10 to 15; and
the fluid channel terminal element (150) comprising a fluid pressure measurement volume (120) with a terminal opening for fluidic coupling to the fluid pressure measurement volume,
wherein the housing element of the component is connectable to the fluid channel terminal element (150) via a snap-on or screw closure.

## Revendications

1. Procédé de fabrication d'un composant d'une unité de mesure de la pression d'un fluide (100) avec un élément de raccordement de canal à fluide (150) présentant un volume de mesure de pression de fluide (120) avec une ouverture de raccordement, aux étapes suivantes consistant à:
préparer un élément de mesure avec une puce de mesure (202) placée sur une première face d'un porte-puce (200) et une pluralité de contacts de raccordement en forme de douille (208) destinés à interconnecter les connexions de contact de la puce de mesure (202), les contacts de raccordement en forme de douille (208) ressortant d'une deuxième face du porte-puce (200) opposée à la première face du porte-puce (200);
préparer un élément de boîtier (216) présentant une pluralité de trous de réception (214) dans une première face de l'élément de boîtier, lesquels sont réalisés de manière à recevoir, lors de l'assemblage de l'élément de boîtier (216) avec l'élément de mesure, les contacts de raccordement en forme de douille (208), les trous de réception (214) s'étendant de la première face de l'élément de boîtier (216) à une deuxième face de l'élément de boîtier (216) opposée à la première face de l'élément de boîtier (216),
la préparation de l'élément de boîtier (216) comprenant une injection directe d'un élément de raccordement (222) à la deuxième face de l'élément de boîtier (216) destiné à obturer les trous de réception (214) dans la deuxième face de l'élément de boîtier (216); et
assembler l'élément de mesure et l'élément de boîtier (216) de sorte que la deuxième face du porte-puce (200) se trouve face à la première face de l'élément de boîtier (216) et que les contacts de raccordement en forme de douille (208) soient disposés dans les trous de réception (214), pour réaliser le composant de l'unité de mesure de pression de fluide (100),
l'ouverture de raccordement étant réalisée de manière à coupler l'élément de mesure ou un gel entourant la puce de mesure avec le volume de mesure de pression de fluide.

2. Procédé selon la revendication 1, dans lequel la préparation de l'élément de boîtier (216) comprend également une injection directe d'un premier élément d'obturation (240) à la première face de l'élément de boîtier (216), l'assemblage de l'élément de mesure et de l'élément de boîtier (216) s'effectuant de sorte que le premier élément d'obturation soit pressé contre la deuxième face du porte-puce (200), pour réaliser le composant de l'unité de mesure de pression de fluide (100).

3. Procédé selon la revendication 2, dans lequel l'étape de préparation de l'élément de boîtier (216) comprend la formation d'une ouverture centrale (220) entre les trous de réception (214), l'ouverture centrale (220) s'étend de la première face de l'élément de boîtier (216) à la deuxième face de l'élément de boîtier (216), l'étape de préparation de l'élément de mesure comprend la préparation d'une ouverture dans le porte-puce (200) au-dessous de la puce de mesure, le premier élément d'obturation (240) est réalisé et l'étape d'assemblage est effectuée de sorte que la pression du premier élément d'obturation contre la deuxième face du porte-puce (200) entraîne une obturation des trous de réception (214) tant par rapport à l'ouverture centrale (220) que par rapport à un espace intérieur (242) qui résulte, lors d'un assemblage de l'élément de raccordement de canal à fluide (150) avec l'élément de boîtier (216), entre l'élément de raccordement de canal à fluide (150) et l'élément de boîtier et que, à l'état assemblé, l'élément de boîtier (216) puisse être raccordé à l'élément de raccordement de canal à fluide (150) de sorte que soit rendue possible une transmission de pression d'un fluide se trouvant dans le volume de mesure de pression de fluide (120), par l'intermédiaire de l'ouverture de raccordement, à la puce de mesure (202).

4. Procédé selon la revendication 2 ou 3, dans lequel l'étape d'injection directe comprend par ailleurs une injection directe d'un deuxième élément d'obturation (228) à la première face de l'élément de boîtier (216), et dans lequel l'assemblage de l'élément de mesure et de l'élément de boîtier (216) est par ailleurs effectué de sorte que, à l'état assemblé, l'élément de boîtier (216) puisse être raccordé à l'élément de raccordement de canal à fluide (150) de sorte que soit rendue possible une transmission de pression d'un fluide se trouvant dans le volume de mesure de pression de fluide (120), par l'intermédiaire de l'ouverture de raccordement, à la puce de mesure (202) et que le deuxième élément d'obturation (228) entraîne, par une compression de l'élément de raccordement de canal à fluide (150) et de l'élément de boîtier (216), un assemblage étanche aux fluides entre ces derniers.

5. Procédé selon l'une des revendications 1 à 4, dans lequel l'étage de préparation de l'élément de mesure comprend un assemblage électrique par brasure des connexions brasées (210) des contacts de raccordement en forme de douille (208) avec les connexions de contact de la puce de mesure (202) et l'assemblage de l'élément de mesure et de l'élément de boîtier (216) n'a lieu qu'après l'assemblage électrique.

6. Procédé de fabrication d'une unité de mesure de la pression d'un fluide, aux étapes consistant à:
réaliser un procédé selon l'une des revendications précédentes;
préparer l'élément de raccordement de canal à fluide (150) présentant un volume de mesure de pression de fluide (120) avec une ouverture de raccordement pour le couplage fluidique avec le volume de mesure de pression de fluide (120); et
assembler le composant de l'unité de mesure de la pression d'un fluide avec l'élément de raccordement de canal à fluide (150) de sorte que soit rendue possible une transmission de pression d'un fluide se trouvant dans le volume de mesure de pression de fluide (120), par l'intermédiaire de l'ouverture de raccordement, à la puce de mesure (202).

7. Procédé selon la revendication 6, dans lequel l'étape d'assemblage du composant de l'unité de mesure de la pression comprend une pression de l'élément de mesure contre l'élément de boîtier (216).

8. Procédé selon la revendication 6 ou 7, dans lequel la préparation de l'élément de raccordement de canal à fluide (150) comprend une préparation d'un canal à fluide (120) présentant, à un endroit à l'emplacement de l'ouverture de raccordement, une section plus petite qu'à un autre endroit, et à l'emplacement de l'ouverture de raccordement étant réalisée une fenêtre d'inspection dans une paroi du canal à fluide, de sorte que soit rendue possible de l'extérieur de l'unité de mesure de pression de fluide (100) une inspection de la puce de mesure (202) ou d'un gel recouvrant le puce de mesure (202).

9. Procédé selon l'une des revendications 6 à 8, avec un procédé dans lequel l'étape de préparation de l'élément de mesure comprend le placement d'un dôme de capteur (204) sur la première face du porte-puce (200), le dôme de capteur (204) entourant la puce de mesure (202) et l'étape de préparation comprenant par ailleurs le remplissage du dôme de capteur (204) avec un gel, de sorte que la puce de mesure (202) soit recouverte par le gel, le remplissage ayant lieu avent l'assemblage.

10. Composant destiné à être utilisé dans une unité de mesure de la pression d'un fluide (100), le composant présentant les caractéristiques suivantes:
un élément de boîtier (216) avec une pluralité de trous de réception (214) s'étendant d'une première face de l'élément de boîtier (216) à une deuxième face de l'élément de boîtier (216), opposée à la première face de l'élément de boîtier, l'élément de boîtier (216) présentant, sur la deuxième face, un élément d'obturation (222) destiné à obturer les ouvertures de réception (214) sur la deuxième face de l'élément de boîtier (216), lequel est assemblé d'une seule pièce avec l'élément de boîtier (216), le matériau de l'élément d'obturation (222) différant d'un matériau de l'élément de boîtier (216); et
un élément de mesure avec une puce de mesure (202) disposée sur une première face d'un porte-puce (200) et une pluralité de contacts de raccordement en forme de douille (208) destinés à interconnecter les connexions de contact de la puce de mesure (202), les contacts de raccordement en forme de douille (208) ressortant d'une deuxième face du porte-puce (200) opposée à la première face du porte-puce (200),
l'élément de mesure et l'élément de boîtier (216) étant conçus de sorte que l'élément de mesure et l'élément de boîtier (216) puissent être assemblés de sorte que, à l'état assemblé, les contacts de raccordement en forme de douille (208) s'engagent dans les trous de réception (214) et que la deuxième face du porte-puce (200) fasse face à la première face de l'élément de boîtier (216), pour former le composant destiné à être utilisé dans l'unité de mesure de pression de fluide (100).

11. Composant selon la revendication 10, dans lequel l'élément de boîtier (216) présente, sur la première face, un premier élément d'obturation (240) qui est assemblé d'une seule pièce avec l'élément de boîtier (216), un matériau du premier élément d'obturation (240) différant d'un matériau de l'élément de boîtier (216), et dans lequel l'élément de mesure et l'élément de boîtier (216) peuvent être assemblés de sorte que, à l'état assemblé, le premier élément d'obturation soit pressé contre la deuxième face du porte-puce (200).

12. Dispositif selon la revendication 11, dans lequel l'élément de boîtier (216) comporte une ouverture centrale (220) entre les trous de réception (214) qui s'étend de la première face de l'élément de boîtier (216) à la deuxième face de l'élément de boîtier (216), l'élément de mesure comporte une ouverture dans le porte-puce (200) au-dessous de la puce de mesure, le premier élément d'obturation (240) est réalisé et l'élément de mesure et l'élément de boîtier (216) peuvent être assemblés de sorte que la pression du premier élément d'obturation contre la deuxième face du porte-puce (200) entraîne une obturation des trous de réception (214) tant par rapport à l'ouverture centrale (220) que par rapport à un espace intérieur (242) qui résulte, lors d'un assemblage de l'élément de raccordement de canal à fluide (150) avec l'élément de boîtier (216), entre l'élément de raccordement de canal à fluide (150) et l'élément de boîtier et que, à l'état assemblé, l'élément de boîtier (216) puisse être assemblé avec l'élément de raccordement de canal à fluide (150) de sorte que soit rendue possible une transmission de pression d'un fluide se trouvant dans le volume de mesure de pression de fluide (120), par l'intermédiaire de l'ouverture de raccordement, à la puce de mesure (202).

13. Composant selon la revendication 12, dans lequel l'élément de mesure et l'élément de boîtier (216) sont en outre conçus de sorte que l'élément de mesure et l'élément de boîtier (216) puissent être assemblés de sorte que, à l'état assemblé, l'élément de boîtier (216) puisse être assemblé avec l'élément de raccordement de canal à fluide (150) de sorte que soit rendue possible une transmission de pression d'un fluide se trouvant dans le volume de mesure de pression de fluide (120), par l'intermédiaire de l'ouverture de raccordement, à la puce de mesure (202) et que le deuxième élément d'obturation (228) entraîne, par une compression de l'élément de raccordement de canal à fluide (150) et de l'élément de boîtier (216), un assemblage étanche aux fluides entre ces derniers.

14. Composant selon l'une des revendications 11 à 13, dans lequel l'élément de boîtier (216) comporte une ouverture centrale (220) entre les trous de réception (214), l'ouverture centrale (220) s'étendant de la première face de l'élément de boîtier (216) à la deuxième face de l'élément de boîtier (216), une ouverture étant prévue dans le porte-puce (200) au-dessous de la puce de mesure, l'ouverture centrale (220) communiquant avec l'ouverture du porte-puce, la puce de mesure (202) étant reliée, sur sa face arrière, par l'intermédiaire de l'ouverture du porte-puce et de l'ouverture centrale, à un milieu extérieur du composant de l'unité de mesure de pression de fluide (100) et les contacts en forme de douille étant, à l'état assemblé, obturés par le premier élément d'obturation (240) par rapport à l'ouverture centrale et un espace intérieur (242) entre l'élément de boîtier (216) et l'élément de raccordement de canal à fluide (150) qui résulte, lors d'un assemblage de l'élément de raccordement de canal à fluide (150), avec l'élément de boîtier (216).

15. Composant selon l'une des revendications 10 à 14, dans lequel l'élément de boîtier (216) présente, par ailleurs, une pluralité de trous d'interconnexion (224) dans l'élément d'obturation à l'endroit des trous de réception (214).

16. Unité de mesure de la pression dans un fluide avec
un composant selon l'une des revendications 10 à 15; et
l'élément de raccordement de canal à fluide (150) présentant un volume de mesure de pression de fluide (120) avec une ouverture de raccordement pour le couplage fluidique avec le volume de mesure de pression de fluide,
l'élément de boîtier du composant pouvant être assemblé par une connexion d'encliquetage ou vissée avec l'élément de raccordement de canal à fluide (150).
